# EUROPEAN PATENT SPECIFICATION

(11) **EP 2 486 879 B1**
(45) Date of publication and mention of the grant of the patent: **03.01.2018**
(21) Application number: 10821797.7
(22) Date of filing: 11.08.2010
(51) Int. Cl.: A61B 17/00

(54) **GUIDE DEVICE**
FÜHRUNGSVORRICHTUNG
DISPOSITIF DE GUIDAGE

(30) Priority: 06.10.2009 JP 2009232468
(43) Date of publication of application: 15.08.2012
(73) Proprietor: Olympus Corporation, Tokyo 192-8507 (JP)
(72) Inventor: SUGAHARA, Michihiro, Hachioji-shi, Tokyo 192-8507 (JP); KOBAYASHI, Masayuki, Hachioji-shi, Tokyo 192-8507 (JP); OKAZAKI, Yoshiro, Hachioji-shi, Tokyo 192-8507 (JP)
(74) Representative: Gunzelmann, Rainer
(86) International application number: PCT/JP2010/063636
(87) International publication number: WO 2011/043126

(56) References cited:
- EP-A1- 0 998 953
- WO-A1-96/40368
- WO-A2-2004/045480
- JP-A- S5 573 235
- JP-A- 55 014 017
- JP-A- 55 073 235
- US-A- 2 907 327
- US-A- 4 662 870
- US-A1- 2009 149 848

## Description

### {Technical Field}

The present invention relates to guide devices.

### {Background Art}

In the related art, there is a known procedure for percutaneously treating the heart in heart surgery by inserting a cardiac treatment instrument that is inserted into the body into the pericardium through a hole formed in the pericardium. The insertion of the cardiac treatment instrument into the pericardium is carried out by, for example, the following steps. Initially, a puncture needle is inserted from below the xiphoid process to perforate the pericardium, thereby placing the tip of the puncture needle in the pericardium. A guide wire is then inserted through the lumen of the puncture needle to place the tip of the guide wire at the target position in the pericardium. The puncture needle is then removed, with the guide wire left in position, a sheath is inserted into the pericardium along the guide wire, and the guide wire is removed, with the sheath left in position. The cardiac treatment instrument can thus be guided through the sheath into the pericardium.

This procedure uses a method in which the puncture needle is introduced into the pericardium while checking its tip position under radioscopy. It is difficult, however, to accurately determine the relative positions of the tip of the puncture needle and the tissue because radioscopic images are two-dimensional. In particular, it is extremely difficult to determine whether or not the tip of the puncture needle has reached the pericardium because the pericardium is invisible in radioscopic images. There are therefore some known devices for detecting that the tip of, for example, a puncture needle has reached the tissue (see, for example, PTLS 1 and 2). In addition, only a small cavity is present between the heart and the pericardium, which surrounds the heart. There are therefore some known devices for selectively puncturing the pericardium (see, for example, PTLS 3 to 5).

### {Citation List}

### {Patent Literature}

{PTL 1} PCT International Publication No. WO 01/078809
{PTL 2} Japanese Unexamined Patent Application, Publication No. 2004-081852
{PTL 3} PCT International Publication No. WO 96/040368
{PTL 4} PCT International Publication No. WO 99/013936
{PTL 5} PCT International Publication No. WO 98/024378

### {Summary of Invention}

### {Technical Problem}

In PTLs 1 and 2, a change in the load on the tip of the puncture needle is detected from extension and compression of a spring to detect that the tip of the puncture needle has contacted the tissue or that the puncture needle has perforated the pericardium. A problem arises, however, in that it is difficult to accurately detect the contact of the puncture needle with the pericardium or the perforation of the pericardium every time because the hardness of the pericardium varies with, for example, the patient and the position.

In PTLs 3 to 5, the pericardium is selectively punctured while expanding the cavity between the heart and the pericardium by attracting or holding part of the pericardium and pulling it outward. It is difficult, however, to advance the devices of PTLs 3 and 4 to the pericardium while cutting the tissue. A problem therefore arises in that an insertion path to the pericardium needs to be formed in advance using another device, thus complicating the procedure.

In addition, when the devices of PTLs 4 and 5 are introduced from the xiphoid process into the pericardium in order to puncture the pericardium, the pericardium is combined with the inner surface of the sternum and the diaphragm near the position reached by the devices; therefore, a problem arises in that pulling the pericardium is insufficient to separate the pericardium from the heart. In addition, these devices need to be disposed perpendicularly to the pericardium. A problem here is that the devices cannot be disposed as such in the body when introduced from the xiphoid process into the pericardium.

US 4 662 870 A concerns a device for indicating the penetration of a needle into a vein, artery, lumen, or other cavity in a patient's body including a filament that is moved through the needle toward its sharpened end. As the needle is inserted through bodily tissue, the distal end of the filament is retained by the tissue at the needle's sharpened end. The filament is loaded with compressive force so that the distal tip is moved through the end of the needle when the end enters a space. Penetration is indicated by the sudden movement of a visible indicator attached to the filament that is caused when the retention of the filament's distal tip is released by the penetration of the needle's end into the space.

US 2 907 327 A concerns an implanting device including a combination cartridge, needle member and a body. A charge of medicinal pellets is packaged within the combination needle and cartridge member and retained therein by means of obstructing means which can either be removed, bypassed or broken by the inserting plunger. A detachable coupling means is incorporated within one end of the combination needle and cartridge member and holder to permit convenient attachment and detachment.

JP S55 73235 A concerns a perforation device having an injection needle with a coil spring and a spiral tube.

Document US 2009/0149848 A1 concerns a solution for the ablation of tissue, wherein an ablation catheter is provided with a deflectable end that can assume a number of different deflection geometries.

An object of the present invention, which has been made in light of the circumstances described above, is to provide a guide device that can be inserted into the body in a simple and easy manner, that can be used by inserting it from the xiphoid process to selectively and easily perforate the pericardium, and that enables reliable detection that it has entered the pericardium.

### {Solution to Problem}

To achieve the above object, the present invention provides the following solutions.

The present invention provides a guide device comprising the features of claim 1.

According to the present invention, the insertion part is inserted into the body, is advanced to the target position while cutting the tissue with the perforating part at the distal end thereof, and is left in position so that another cardiac treatment instrument can be guided through the insertion part into the pericardium. In this case, the moving part is advanced through the body while being held at the nearby position against the biasing force of the biasing mechanism as the moving part is pressed by the body tissue. Upon entering the cavity, the moving part is released from being pressed by the tissue and is displaced to the farther position.

That is, it is possible to detect the displacement of the moving part with the displacement-detecting mechanism to reliably and quickly detect that the distal end of the insertion part has entered the pericardium, and it is also possible to stop further insertion of the insertion part to selectively perforate the pericardium. In addition, the insertion part can be easily advanced while cutting the body tissue with the perforating part disposed at the distal end of the insertion part, and the pericardium can be easily perforated by inserting the insertion part from the xiphoid process.

In the above invention, the perforating part may be a sharp tip part, a drill, dissecting forceps, or an electric knife.

This facilitates tissue cutting and pericardium perforation.

In the above invention, the moving part may be disposed so as to be movable backwards and forwards in a longitudinal direction of the insertion part, and the biasing mechanism may bias the moving part in a direction away from the distal end of the insertion part in the longitudinal direction of the insertion part.

This simplifies the structure of the moving part.

In this configuration, the moving part may include a directing mechanism that directs the distal end of the moving part in a direction crossing the longitudinal direction as the moving part projects in the longitudinal direction.

Thus, the orientation of the moving part is changed, thereby allowing the displacement thereof to be more easily detected and preventing the moving part from contacting the tissue present in the cavity in the forward direction.

In the above invention, the moving part may be disposed so as to emerge radially from the side of the insertion part, and the biasing mechanism may bias the moving part outward in the radial direction of the insertion part.

In the above invention, the displacement-detecting mechanism may include a wire joined to the moving part and having a mark disposed at a position farther away from the proximal side of the insertion part outside the insertion part.

Thus, the mark is moved forward relative to the insertion part as the moving part pulls the tip of the wire when the moving part is displaced from the nearby position to the farther position. This allows the movement of the moving part to be easily detected outside the patient's body.

In the above invention, the moving part may include a balloon that is expandable and contractible in the radial direction of the insertion part, and the biasing mechanism may pressurize the interior of the balloon.

Thus, upon entering the pericardium, the balloon expands as it is released from being pressed by the tissue. Thus, it is possible to easily detect that the distal end of the insertion part has entered the pericardium. In addition, the operator can easily contract the balloon by stopping the pressurization of the interior of the balloon as needed.

In this configuration, the displacement-detecting mechanism may be a radiocontrast agent that is supplied into the balloon, and the biasing mechanism may pressurize the radiocontrast agent.

Thus, the position and expansion of the balloon can be easily visually recognized in a radioscopic image.

In the above invention, the displacement-detecting mechanism may be composed of at least a portion of the moving part, and the portion may be formed of a radio-opaque material.

Thus, the position and displacement of the moving part in the body can be easily visually recognized in a radioscopic image.

In the above invention, the guide device may have a conduit formed in the longitudinal direction of the insertion part and having an orifice that opens near the distal end of the insertion part and an injection port that opens on the proximal side of the insertion part.

Thus, a radiocontrast agent is injected from the injection port into the conduit and is thereby ejected from the orifice at the distal end of the insertion part into the body. This allows the distal end of the insertion part in the body to be more reliably located.

### {Advantageous Effects of Invention}

The present invention provides an advantage in that the guide device can be inserted into the body in a simple and easy manner, can be used by inserting it from the xiphoid process to selectively and easily perforate the pericardium, and enables reliable detection that it has entered the pericardium. The invention is as disclosed in the appended set of claims.

### {Brief Description of Drawings}

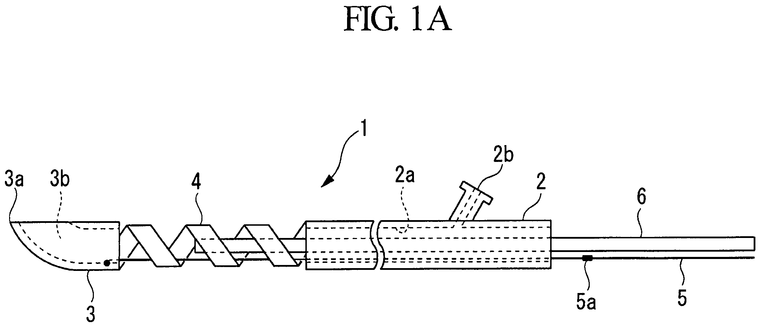

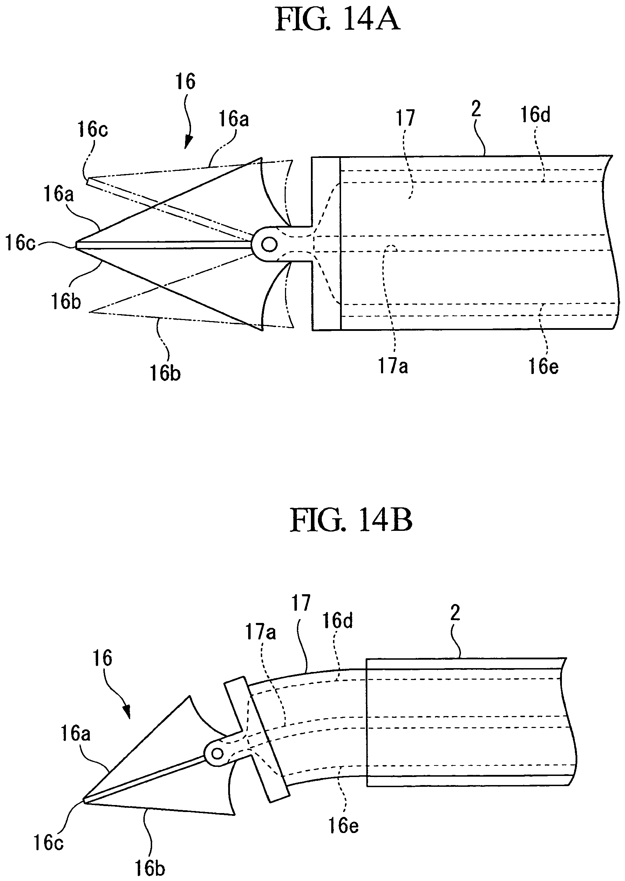

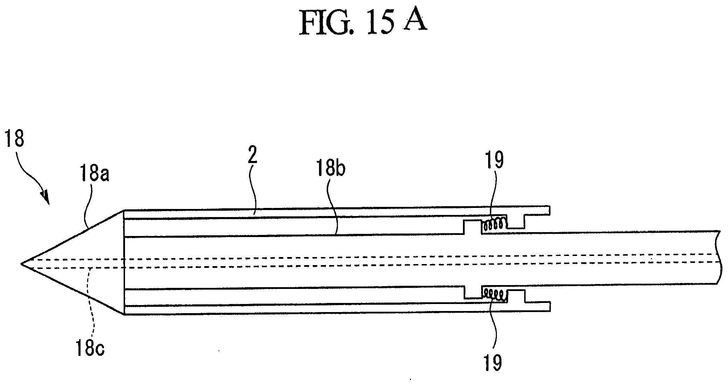

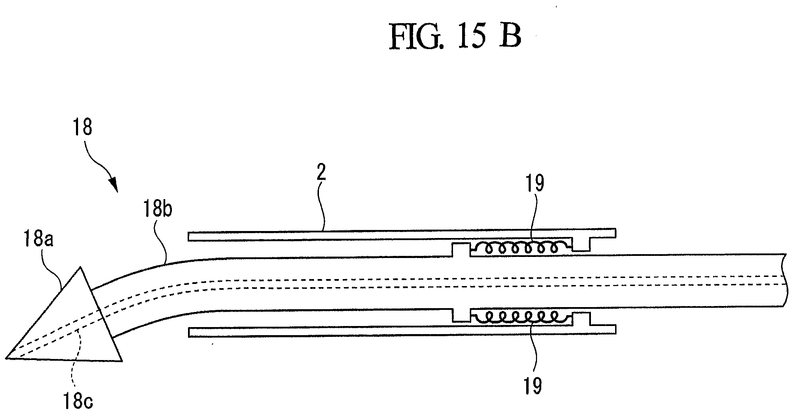

{Fig. 1A}
   Fig. 1A is a general schematic view of a guide device according to an embodiment of the present invention, showing the state where a spring is extended to its natural state.
{Fig. 1B}
   Fig. 1B illustrates the guide device in Fig. 1A, showing the state where the spring is compressed.
{Fig. 2A}
   Fig. 2A illustrates a method for using the guide device in Fig. 1A, showing the state when it is inserted to the vicinity of the heart.
{Fig. 2B}
   Fig. 2B illustrates the method for using the guide device in Fig. 1A, showing the state when it is further advanced to the vicinity of the heart.
{Fig. 2C}
   Fig. 2C illustrates the method for using the guide device in Fig. 1A, showing the state after it perforates the pericardium.
{Fig. 3A}
   Fig. 3A illustrates a modification of a biasing mechanism in Fig. 1A, showing the state where an elastic member is compressed.
{Fig. 3B}
   Fig. 3B illustrates the biasing mechanism in Fig. 3A, showing the state where the elastic member is released from an external force.
{Fig. 4A}
   Fig. 4A illustrates a modification of a moving part in Fig. 1A, showing the state where vane-shaped portions are spread out.
{Fig. 4B}
   Fig. 4B illustrates the moving part in Fig. 4A, showing the state where the vane-shaped portions are contained while being pressed by the body tissue.
{Fig. 5}
   Fig. 5 illustrates a modification of the vane-shaped portions in Fig. 4A, having manipulating wires connected thereto.
{Fig. 6}
   Fig. 6 illustrates an example of a method for removing the guide device in Fig. 4A.
{Fig. 7}
   Fig. 7 illustrates bar-shaped members serving as another modification of the moving part in Fig. 1A.
{Fig. 8}
   Fig. 8 illustrates a coil spring serving as another modification of the moving part in Fig. 1A.
{Fig. 9}
   Fig. 9 illustrates a balloon serving as another modification of the moving part in Fig. 1A.
{Fig. 10}
   Fig. 10 illustrates another modification of the guide device in Fig. 1A.
{Fig. 11}
   Fig. 11 illustrates another modification of the guide device in Fig. 1A.
{Fig. 12A}
   Fig. 12A illustrates another modification of the guide device in Fig. 1A, showing the state where a distal part projects forward.
{Fig. 12B}
   Fig. 12B illustrates the guide device in Fig. 12A, showing the state where the distal part is in close contact with the insertion part.
{Fig. 13A}
   Fig. 13A illustrates a modification of the insertion part and the distal part in Figs. 12A and 12B.
{Fig. 13B}
   Fig. 13B illustrates another modification of the insertion part and the distal part in Figs. 12A and 12B.
{Fig. 14A}
   Fig. 14A illustrates an electric knife serving as a modification of a perforating part of the guide device in Fig. 1A, showing the state where a soft part is compressed and contained in the insertion part.
{Fig. 14B}
   Fig. 14A illustrates the electric knife in Fig. 14A, showing the state where the soft part is released from an external force.
{Fig. 15A}
   Fig. 15A illustrates a drill serving as a modification of the perforating part of the guide device in Fig. 1A, showing the state where springs are compressed.
{Fig. 15B}
   Fig. 15B illustrates the drill in Fig. 15A, showing the state where the springs are released from an external force. Figures 14A, 14B, 15A and 15B are not part of the invention.

### {Description of Embodiments}

A guide device 1 according to an embodiment of the present invention will be described below with reference to the drawings.

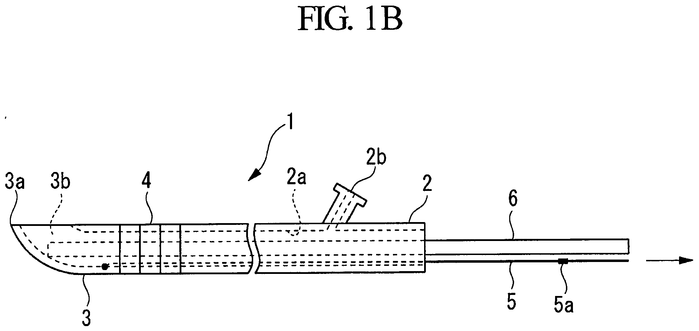

As shown in Figs. 1A and 1B, the guide device 1 according to this embodiment includes a cylindrical insertion part 2, a distal part (moving portion) 3 disposed on the distal side of the insertion part 2, a spring (biasing mechanism) 4 disposed between the insertion part 2 and the distal part 3, a manipulating wire (displacement-detecting mechanism, wire) 5 for moving the distal part 3 backwards and forwards, and a core bar 6 that can be inserted into and retracted from the insertion part 2.

The insertion part 2 is formed of a metal having a relatively small biological effect or a resin such as urethane, PTFE, or Duracon. The insertion part 2 has a lumen (conduit) 2a passing therethrough in the longitudinal direction. The insertion part 2 has an injection port 2b leading to the lumen 2a on the proximal side thereof. A contrast agent is injected into the lumen 2a through, for example, a syringe connected to the injection port 2b and is thereby ejected from an opening (orifice) at the distal end of the insertion part 2.

The distal part 3 is a cylinder that opens at each end thereof and that is curved in one direction from the longitudinal direction of the insertion part 2. The distal part 3 has a sharp tip, perforating part) 3a at the distal end thereof. At least a portion of the distal part 3 is formed of a radio-opaque material through which no X-rays pass (displacement-detecting mechanism) so that its position in the body can be easily visually recognized under radioscopy. The distal part 3 has a through-hole 3b leading to the lumen 2a through the air core of the spring 4.

The spring 4 is formed in a coil shape. In a natural state free of a longitudinal external force, as shown in Fig. 1A, the spring 4 is extended so that it bends flexibly along the shape of the body tissue. The spring 4 has a sufficiently low spring constant; as shown in Fig. 1B, it can be compressed against its elastic force when pressed against the body tissue.

The manipulating wire 5 has its tip fixed to the distal part 3 and extends through the air core of the spring 4 and the lumen 2a outside the proximal end surface of the insertion part 2. When the operator manually pulls the manipulating wire 5, as shown in Fig. 1B, the spring 4 is compressed so that the distal part 3 approaches the distal end surface of the insertion part 2, with the spring 4 being stressed. This provides the entire cardiac guide device 1 with sufficient stiffness for it to be advanced while perforating the body tissue with the sharp tip 3a.

The manipulating wire 5 has a mark (displacement-detecting mechanism) 5a outside the insertion part 2. This allows the operator to easily check the extension/compression state of the spring 4 from the length of the manipulating wire 5 from the proximal end surface of the insertion part 2 to the mark 5a without having to externally visually recognize the state of the spring 4 inserted into the body.

The method for using the thus-configured guide device 1 according to this embodiment and the operation thereof will be described below.

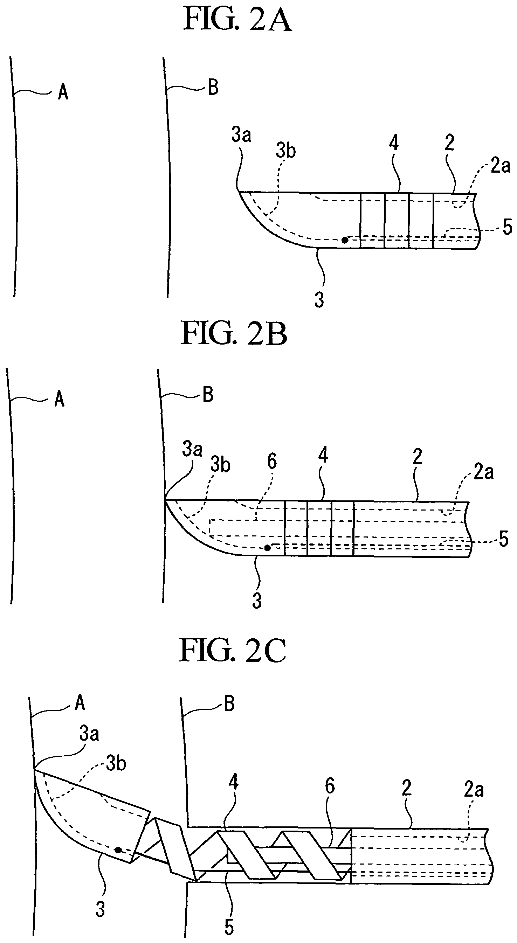

The guide device 1 according to this embodiment is inserted into the body before the insertion of a guide wire for guiding another cardiac treatment instrument to the target position. As shown in Fig. 2A, the operator inserts the guide device 1 from below the patient's xiphoid process, with the manipulating wire 5 being pulled. The operator then advances the distal part 3 toward a heart A while checking the position of the distal part 3 in a radioscopic image. During advancement, the tissue is easily perforated with the sharp tip 3a provided on the distal part 3.

After bringing the distal part 3 into the vicinity of the heart A, as shown in Fig. 2B, the operator inserts the core bar 6 into the insertion part 2 and pushes the distal part 3 with the core bar 6 to gradually advance the distal part 3 forward. As shown in Fig. 2C, the operator determines that the distal part 3 has projected forward as the spring 4 extends in a radioscopic image and from the movement of the mark 5a. The guide device 1 is then inserted slightly farther away from that position, and the core bar 6 is withdrawn, with the guide device 1 left in position. A radiocontrast agent is then injected into the lumen 2a through, for example, a syringe connected to the injection port 2b, and it is checked whether the radiocontrast agent diffuses along the inner shape of a pericardium B.

Next, a guide wire is inserted through the lumen 2a. The tip of the guide wire, emerging from the distal end surface of the distal part 3, is inserted to the target position while checking it in a radioscopic image. The guide device 1 is then removed from the body, with the guide wire left in position. Subsequently, a cardiac treatment instrument such as a guide sheath or an endoscope is guided from below the xiphoid process to the target position in the pericardium B by inserting it along the guide wire.

Thus, according to the present invention, upon perforating the pericardium B, the distal part 3 is released from the pressing force exerted by the surrounding tissue or the pericardium B and projects forward as the spring 4 extends to its natural state. This provides the advantage of quickly and reliably detecting that the guide device 1 has perforated and entered the pericardium B, thus enabling the pericardium B to be selectively perforated. Another advantage is that the flexibility of the spring 4 effectively buffers the impact of the sharp tip 3a on the heart A when the sharp tip 3a enters the pericardium B in the above manner and contacts the heart A. A further advantage is that the spring 4 is compressed to a highly stiff state as the guide device 1 is inserted so that it can be easily inserted while cutting the body tissue.

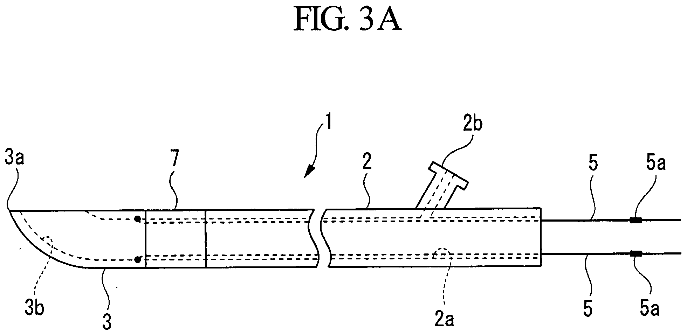

While the spring 4 is provided between the insertion part 2 and the distal part 3 in the above embodiment, as shown in Fig. 3A, a cylindrical elastic member (biasing mechanism) 7 may be provided instead. The material used for the elastic member 7 is, for example, a resin such as polyurethane or silicone, or rubber. Thus, the same advantages as in the above embodiment can be provided by pulling the manipulating wire 5 to stress the elastic member 7, thereby increasing its stiffness, and by the elastic member 7 extending upon entering the pericardium B.

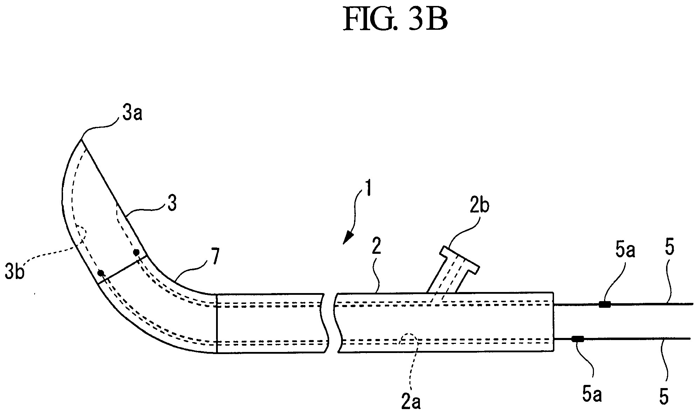

In this case, as shown in Fig. 3B, the elastic member (directing mechanism) 7 may be formed so as to be curved in its natural state in the same direction as the distal part 3. Thus, the elastic member 7, which is straight before the guide device 1 enters the pericardium B, deforms into a curved shape thereafter. This deformation of the elastic member 7 allows the displacement of the elastic member 7 to be more easily visually recognized and also prevents the distal part 3 from contacting the heart A in the forward direction.

In this case, additionally, the manipulating wire 5 may be joined to each of the inner and outer sides of the curved shape of the elastic member 7. Thus, the marks 5a on the inner and outer sides of the curve are moved by different distances as the elastic member 7 deforms from the straight shape into the curved shape. Accordingly, the deformation of the elastic member 7 can be detected from the change in the relative positions of the marks 5a.

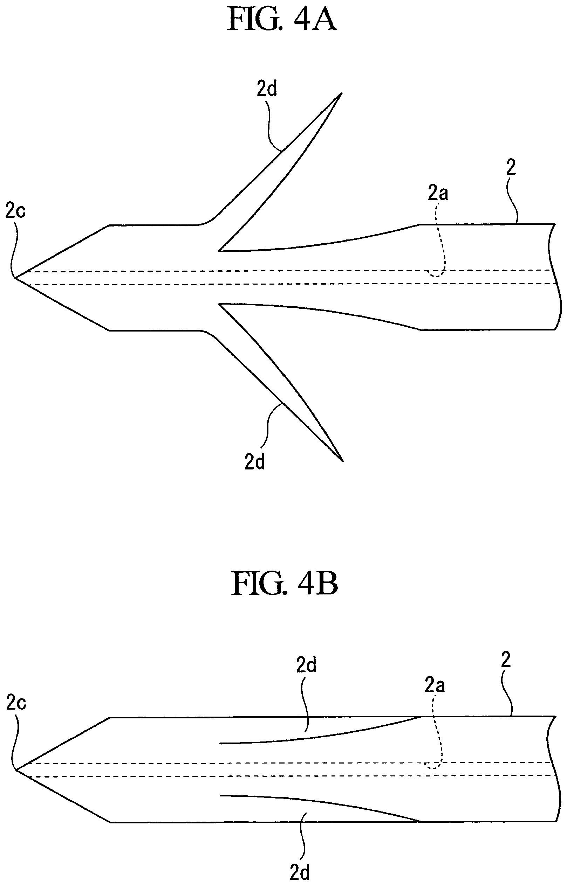

While the guide device 1 has the distal part 3 and the spring 4 at the distal end of the insertion part 2 in the above embodiment, another structure movable in response to a change in external pressure may be provided instead. Examples thereof are shown in Figs. 4A and 4B to Fig. 9.

In the example shown in Fig. 4A, at least the distal portion of the insertion part 2 is formed of a radio-opaque resin (displacement-detecting mechanism). The tip (perforating part) 2c of the insertion part 2 is formed in a needle shape so that it can perforate the tissue, including the pericardium B. Vane-shaped portions (moving part) 2d are provided on the sidewall of the distal portion of the insertion part 2. The vane-shaped portions 2d are formed by cutting the sidewall in a direction along the surface thereof and bending the cut portions so as to protrude radially outward.

Thus, as shown in Fig. 4B, the vane-shaped portions 2d are pressed by the surrounding tissue and are contained in the side of the insertion part 2 as the insertion part 2 is inserted into the body. On the other hand, as shown in Fig. 4A, in the pericardium B, where the vane-shaped portions 2d are released from being pressed by the tissue, they spread out radially into the bent shape.

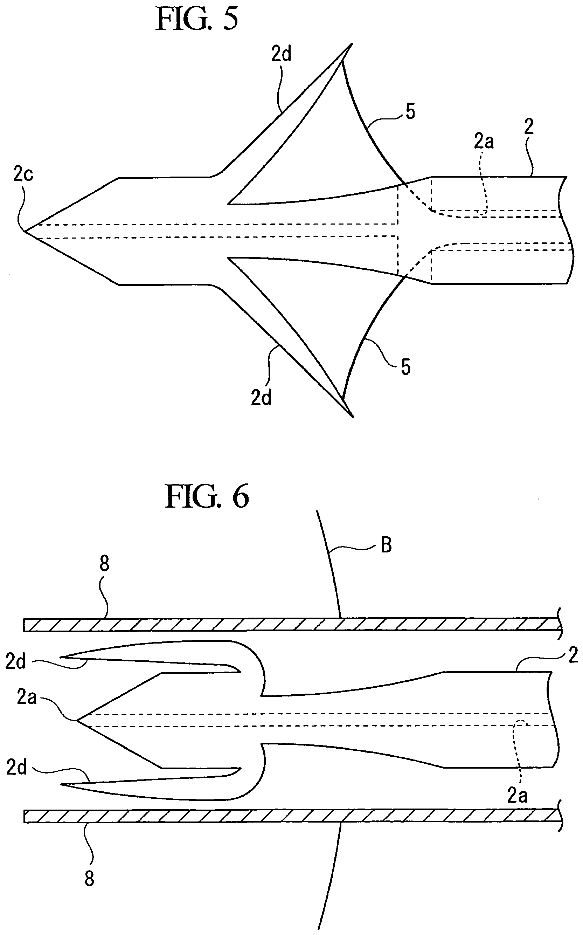

In this case, as shown in Fig. 5, the manipulating wire 5 may be joined to each vane-shaped portion 2d. Thus, the mark provided on the manipulating wire 5 is moved forward when the distal portion of the insertion part 2 enters the pericardium B. Thus, it is possible to more reliably determine that the vane-shaped portions 2d have spread out.

To remove the insertion part 2 having the vane-shaped portions 2d spread out in the pericardium B, as shown in Fig. 4B, from the body, as shown in Fig. 6, an outer sheath 8 having an inner diameter larger than the outer diameter of the insertion part 2 is inserted into the pericardium B along the insertion part 2. The vane-shaped portions 2d are then pushed toward the distal side by the distal end surface of the outer sheath 8 and are contained in the outer sheath 8 while gathering radially. This allows the guide device 1 to be easily removed from the body, with the distal portion of the insertion part 2 contained in the outer sheath 8.

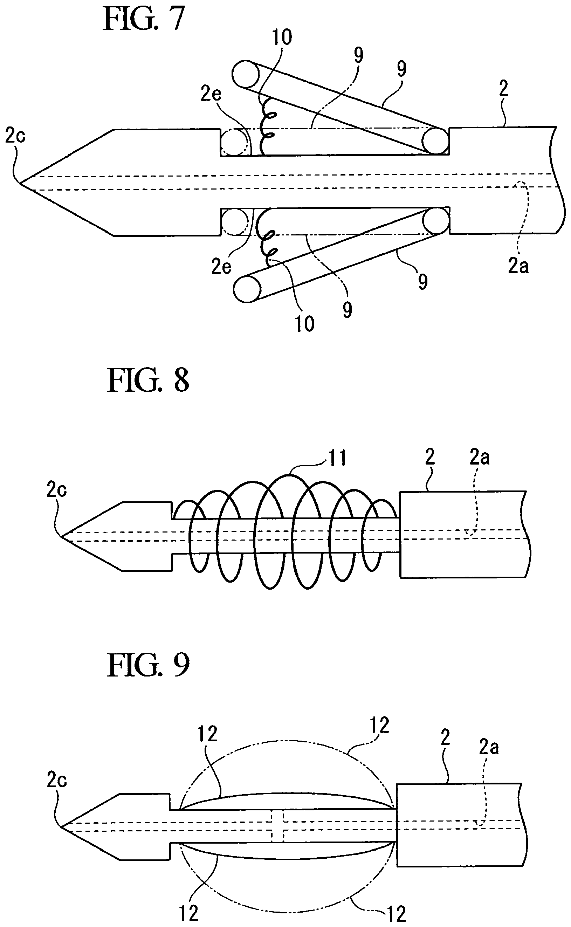

The example shown in Fig. 7 has grooves 2e formed in the sidewall of the distal portion of the insertion part 2 in the longitudinal direction thereof and bar-shaped members (moving part) 9 that can be contained in the grooves 2e. The bar-shaped members 9 are attached at one end to end surfaces of the grooves 2e and are biased at the other end in a direction in which they emerge from the grooves 2e by a biasing mechanism such as springs 10. The bar-shaped members 9 are formed of a radio-opaque material (displacement-detecting mechanism). Thus, as with the vane-shaped portions 2d described above, it is possible to detect that the distal portion of the insertion part 2 has entered the pericardium B when the bar-shaped members 9 emerge radially.

The example shown in Fig. 8 has a coil spring (moving part) 11 spirally coiled around the distal portion of the insertion part 2 in the longitudinal direction thereof. The coil spring 11 protrudes radially from the insertion part 2 when it is free of an external force. When pressed radially, on the other hand, the coil spring 11 is compressed radially to substantially the same size as the insertion part 2. In addition, the coil spring 11 is formed of a radio-opaque material (displacement-detecting mechanism). Thus, it is possible to detect that the distal portion of the insertion part 2 has entered the pericardium B when the coil spring 11 expands radially.

The example shown in Fig. 9 has a balloon (moving part) 12 on the distal portion of the insertion part 2. The balloon 12 communicates with the lumen 2a of the insertion part 2. A radiocontrast agent is injected into the lumen 2a and is pressurized at a pressure substantially equal to the external pressure in the body while the insertion part 2 is advanced in the body. Thus, upon entering the pericardium B, the balloon 12 expands as the pressure at which the radiocontrast agent is pressurized exceeds the external pressure. Thus, it is possible to detect that the distal portion of the insertion part 2 has entered the pericardium B by visually recognizing the expansion of the balloon 12 in a radioscopic image.

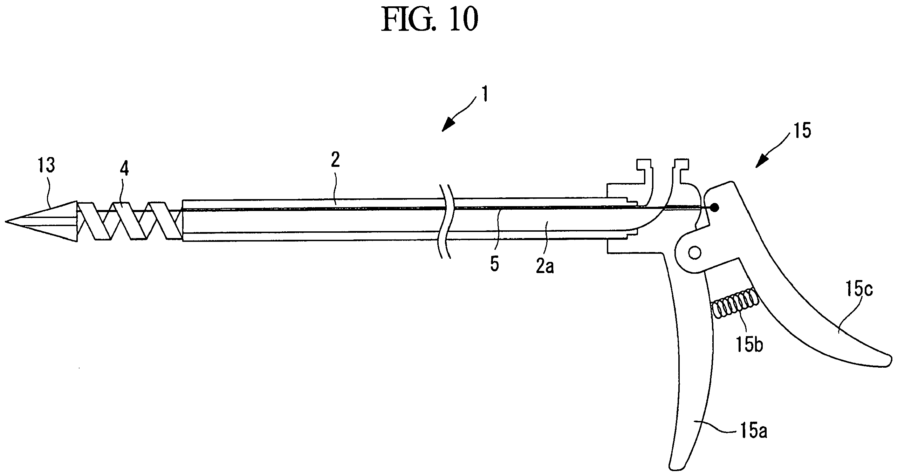

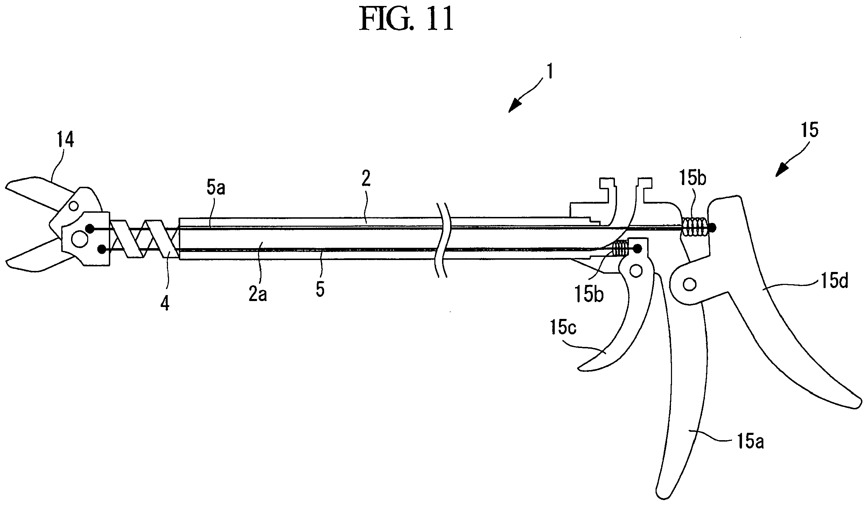

While the cylindrical distal part 3 is disposed on the distal side of the insertion part 2 in the above embodiment, as shown in Fig. 10, a conical distal part 13 having a sharp tip facing the distal side may be provided instead, or as shown in Fig. 11, dissecting forceps (perforating part) 14 may be provided instead. In addition, a manipulating part 15 for manipulating the manipulating wire 5 may be disposed on the proximal side of the insertion part 2.

The manipulating part 15 includes a grip 15a secured to the insertion part 2 and a lever 15c joined to the grip 15a with a lever spring 15b therebetween, and the manipulating wire 5 is joined to the lever 15c. If the dissecting forceps 14 are provided, another lever 15d to which a forceps wire 5a for manipulating the dissecting forceps 14 is joined is provided. The lever springs 15b bias the respective levers 15c and 15d in a direction away from the grip 15a so that the spring 4 is in its natural state or the dissecting forceps 14 are open when the operator does not manipulate the manipulating part 15.

When the operator grips the grip 15a and the lever 15c, the lever 15c is moved in a direction approaching the grip 15a to pull the manipulating wire 5. In addition, when the operator grips the grip 15a and the other lever 15d, the forceps wire 5a is pulled to close the dissecting forceps 14. Thus, it is possible to improve the ease of manipulation of the guide device 1 by the operator and to facilitate tissue cutting and perforation.

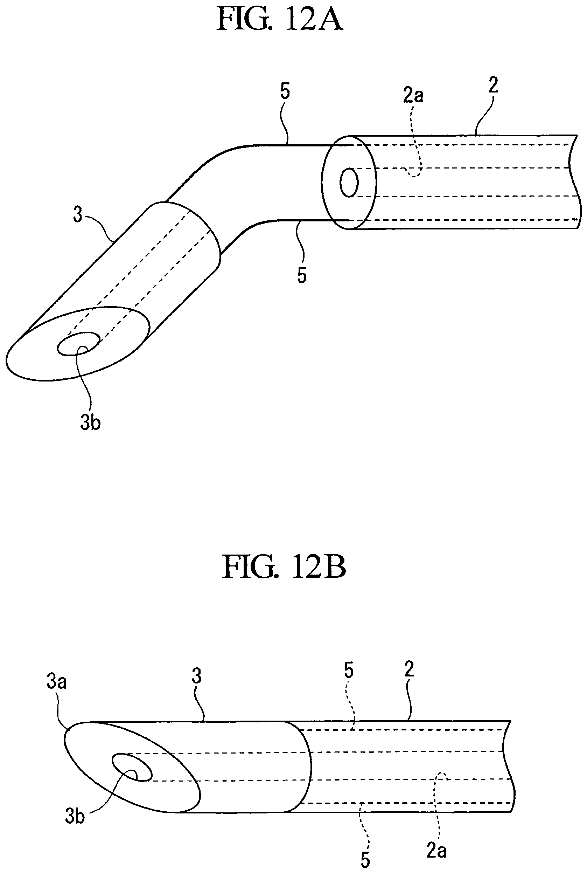

In the above embodiment, as shown in Figs. 12A and 12B, a plurality of (in the example shown, two) manipulating wires 5 may be arranged at substantially regular intervals in the circumferential direction of the insertion part 2. Thus, the individual manipulating wires 5 can be pushed and pulled by
equal forces to manipulate the distal part 3 while stabilizing its position, and one of the manipulating wires 5 can be manipulated to easily curve the distal part 3 in the intended direction.

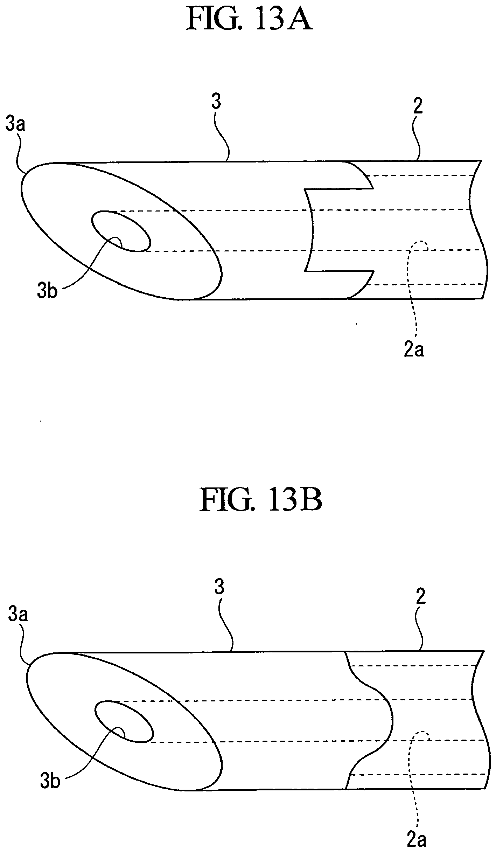

In this case, the distal part 3 may be disposed on the distal side of the insertion part 2 without the spring 4 therebetween. It is then preferable that the proximal end surface of the distal part 3 and the distal end surface of the insertion part 2 be shaped such that they fit with each other. For example, each end surface may be stepped, as shown in Fig. 13A, or may be wavy, as shown in Fig. 13B. Thus, the radial position of the distal part 3 is restricted relative to the insertion part 2 while the distal part 3 is fitted with the insertion part 2 in close contact. This prevents the distal part 3 from buckling under a relatively large external force pressing the distal part 3 in the longitudinal direction, thus stabilizing the position of the distal part 3.

In the above aspect, as shown in Figs. 14A and 14B, the distal part 3 may be replaced by an electric knife (perforating part, moving part) 16. The insertion part 2 contains an elastic soft part (biasing mechanism) 17, with the electric knife 16 disposed at the distal end of the soft part 17. Thus, as shown in Fig. 14A, the entire soft part 17 is compressed into the insertion part 2 as the insertion part 2 is inserted into the body and is pressed by the surrounding tissue. The soft part 17 has a through-hole 17a through which a guide wire can be inserted substantially along the central axis thereof.

The electric knife 16 has, for example, a bipolar configuration including an active electrode 16a and a return electrode 16b. An insulator 16c covers the inner surface of the active electrode 16a to insulate the electrodes 16a and 16b from each other. The electrodes 16a and 16b are connected to conductors 16d and 16e, respectively, extending from the proximal end of the insertion part 2 to the outside. A high-frequency current can be supplied through the conductor 16d to the active electrode 16a to cut the tissue with the tip of the electric knife 16.

In addition, the electrodes 16a and 16b of the electric knife 16 can be opened and closed by manipulating a wire (not shown) on the proximal side of the insertion part 2, as indicated by the chain double-dashed lines in Fig. 14A, thus also serving as dissecting forceps. With the electrodes 16a and 16b open, a guide wire inserted through the through-hole 17a emerges from between the electrodes 16a and 16b.

To advance the electric knife 16 to the vicinity of the heart A, for example, a high-frequency current is applied to cut the tissue. In the vicinity of the heart A, the application of the high-frequency current is stopped, and the electric knife 16 is advanced while gradually dissecting the tissue. When the electric knife 16 perforates the pericardium B, as shown in Fig. 14B, the soft part 17 compressed by pressing extends so that the electric knife 16 projects forward.

In the above aspect, as shown in Figs. 15A and 15B, the distal part 3 may be replaced by a drill (perforating part, moving part) 18.

The drill 18 includes a conical distal part 18a formed of a radio-opaque material and having a sharp tip (perforating part) facing the distal side and a rotating part 18b contained in a sheath 2. The rotating part 18b is connected to a rotating device (not shown) on the proximal side thereof so that it can be rotated in the circumferential direction thereof. Preferably, at least the distal portion of the rotating part 18b is so flexible that it can be curved along the shape of the body tissue while transmitting the torque of the rotating device to the distal portion.

Springs (biasing mechanism) 19 join together the inner surface of the sheath 2 and the side surface of the rotating part 18b at some locations therealong. The springs 19 bias the drill 18 toward the distal side. This causes the distal part 18a of the drill 18 to project from the sheath 2, as shown in Fig. 15B, when it is free of an external force. The distal part 18a and the rotating part 18b have a through-hole 18c through which a guide wire is inserted substantially along the central axis thereof.

Thus, it is possible to easily perforate the tissue and to easily detect that the distal end of the guide device 1 has perforated and entered the pericardium B.

### {Reference Signs List}

- 1: guide device
- 2: insertion part
- 2a: lumen
- 2b: injection port
- 2c: sharp tip (perforating part)
- 2d: vane-shaped portion (moving part)
- 2e: groove
- 3: distal part (moving part)
- 3a: sharp tip (sharp tip part, perforating part)
- 3b: through-hole
- 4: spring (biasing mechanism)
- 5: manipulating wire (wire)
- 5a: mark (displacement-detecting mechanism)
- 6: core bar
- 7: elastic member (biasing mechanism)
- 8: outer sheath
- 9: bar-shaped member (moving part)
- 10, 15b, 19: spring (biasing mechanism)
- 11: coil spring (moving part)
- 12: balloon (moving part)
- 13: distal part (moving part)
- 14: dissecting forceps (moving part)
- 15: manipulating part
- 15a: grip
- 15c, 15d: lever
- 16: electric knife (perforating part, moving part)
- 16a: active electrode
- 16b: return electrode
- 16c: insulator
- 16d, 16e: conductor
- 17: soft part (biasing mechanism)
- 17a: through-hole
- 18: drill (perforating part, moving part)
- 18a: distal part
- 18b: rotating part
- 18c: through-hole
- A: heart
- B: pericardium

## Claims

1. A guide device (1) comprising:
a cylindrical insertion part (2) configured to be inserted into a body and that opens near a distal end thereof and on a proximal side thereof;
a moving part (3) disposed on a distal side of the insertion part (2) and configured to be displaceable between a nearby position near the insertion part (2) and a farther position farther away from the insertion part (2) than the nearby position;
a biasing mechanism (4) having a proximal end mounted to the insertion part (2) and a distal end mounted to the moving part (3), and configured to bias the moving part (3) in a direction away from the insertion part (2); and
a displacement-detecting mechanism (5a) configured to detect displacement of the moving part (3) in the body,
**characterized by**
a perforating part (3a) provided on a distal end of the moving part (3) and configured to perforate pericardial tissue, wherein
the biasing mechanism (4) has a spring constant so that the biasing mechanism (4) can be compressed against its elastic force when the moving part is pressed against a body tissue.

2. The guide device (1) according to Claim 1, wherein the perforating part is a sharp tip part.

3. The guide device (1) according to Claim 1, wherein the perforating part is a drill.

4. The guide device (1) according to Claim 1, wherein the perforating part is dissecting forceps.

5. The guide device (1) according to Claim 1, wherein the perforating part is an electric knife.

6. The guide device (1) according to Claim 1, wherein the moving part (3) is disposed so as to be movable backwards and forwards in a longitudinal direction of the insertion part (2),
the biasing mechanism (4) is configured to bias the moving part (3) in a direction away from the distal end of the insertion part (2) in the longitudinal direction of the insertion part (2).

7. The guide device (1) according to Claim 6, wherein the moving part (3) comprises a directing mechanism configured to direct the distal end of the moving part (3) in a direction crossing the longitudinal direction as the moving part (3) projects in the longitudinal direction.

8. The guide device (1) according to Claim 1, wherein the displacement-detecting mechanism (5a) comprises a wire joined to the moving part (3) and having a mark disposed at a position farther away from the proximal side of the insertion part (2) outside the insertion part (2).

9. The guide device (1) according to Claim 1, wherein the displacement-detecting mechanism (5a) comprises at least a portion of the moving part (3), the portion comprising a radio-opaque material.

10. The guide device (1) according to Claim 1, further comprising a conduit formed in the longitudinal direction of the insertion part (2) and having an orifice that opens near the distal end of the insertion part (2) and an injection port that opens on the proximal side of the insertion part (2).

## Patentansprüche

1. Führungsvorrichtung (1), umfassend:
ein zylinderförmiges Einführteil (2), das ausgestaltet ist, um in einen Körper eingeführt zu werden und das sich in der Nähe eines distalen Endes davon und auf einer proximalen Seite davon öffnet;
ein bewegliches Teil (3), das auf einer distalen Seite des Einführteils (2) angeordnet ist und das ausgestaltet ist, um zwischen einer nahegelegenen Position in der Nähe des Einführteils (2) und einer entfernteren Position, die von dem Einführteil (2) weiter entfernt ist als die nahegelegene Position, verschiebbar ist;
einen Vorspannmechanismus (4), der ein proximales Ende, das an dem Einführteil (2) angebracht ist, und ein distales Ende, das an dem beweglichen Teil (3) angebracht ist, aufweist und das ausgestaltet ist, um das bewegliche Teil (3) in eine Richtung von dem Einführteil weg (2) vorzuspannen; und
einen Verschiebungserkennungsmechanismus (5a), der ausgestaltet ist, um eine Verschiebung des beweglichen Teils (3) in dem Körper zu erkennen,
**gekennzeichnet durch**
ein perforierendes Teil (3a), das an einem distalen Ende des beweglichen Teils (3) bereitgestellt ist und das ausgestaltet ist, um Perikardgewebe zu perforieren, wobei
der Vorspannmechanismus (4) eine Federkonstante aufweist, sodass der Vorspannmechanismus (4) gegen dessen elastische Kraft gepresst werden kann, wenn das bewegliche Teil gegen ein Körpergewebe gedrückt wird.

2. Führungsvorrichtung (1) nach Anspruch 1, wobei das perforierende Teil ein Teil mit einer scharfen Spitze ist.

3. Führungsvorrichtung (1) nach Anspruch 1, wobei das perforierende Teil ein Bohrer ist.

4. Führungsvorrichtung (1) nach Anspruch 1, wobei das perforierende Teil eine Dissektionspinzette ist.

5. Führungsvorrichtung (1) nach Anspruch 1, wobei das perforierende Teil ein elektrisches Messer ist.

6. Führungsvorrichtung (1) nach Anspruch 1, wobei das bewegliche Teil (3) derart angeordnet ist, dass es nach hinten und nach vorne in eine Längsrichtung des Einführteils (2) beweglich ist,
der Vorspannmechanismus (4) ausgestaltet ist, um das bewegliche Teil (3) in eine Richtung von dem distalen Ende des Einführteils (2) weg in Längsrichtung des Einführteils (2) vorzuspannen.

7. Führungsvorrichtung (1) nach Anspruch 6, wobei das bewegliche Teil (3) einen Lenkungsmechanismus umfasst, der ausgestaltet ist, um das distale Ende des beweglichen Teils (3) in eine die Längsrichtung kreuzende Richtung zu lenken, wenn das bewegliche Teil (3) in Längsrichtung hervorsteht.

8. Führungsvorrichtung (1) nach Anspruch 1, wobei der Verschiebungserkennungsmechanismus (5a) einen Draht umfasst, der mit dem beweglichen Teil (3) verbunden ist und der eine Markierung aufweist, die an einer Position angeordnet ist, die weiter von der proximalen Seite des Einführteils (2) außerhalb des Einführteils (2) entfernt angeordnet ist.

9. Führungsvorrichtung (1) nach Anspruch 1, wobei der Verschiebungserkennungsmechanismus (5a) mindestens einen Abschnitt des beweglichen Teils (3) umfasst, wobei der Abschnitt ein strahlenundurchlässiges Material umfasst.

10. Führungsvorrichtung (1) nach Anspruch 1, ferner umfassend eine Leitung, die in Längsrichtung des Einführteils (2) ausgebildet ist und die eine Öffnung, die sich in der Nähe des distalen Endes des Einführteils (2) öffnet, und einen Injektionsport aufweist, der sich an der proximalen Seite des Einführteils (2) öffnet.

## Revendications

1. Dispositif de guidage (1) comprenant :
une partie d'insertion cylindrique (2) configurée pour être insérée dans un corps et qui s'ouvre près d'une extrémité distale de celle-ci et sur un côté proximal de celle-ci ;
une partie mobile (3) disposée sur un côté distal de la partie d'insertion (2) et configurée pour pouvoir se déplacer entre une position proche, qui est proche de la partie d'insertion (2), et une position plus éloignée, qui est plus éloignée de la partie d'insertion (2) que la position proche ;
un mécanisme de sollicitation (4) ayant une extrémité proximale montée sur la partie d'insertion (2) et une extrémité distale montée sur la partie mobile (3), et configuré pour solliciter la partie mobile (3) dans une direction s'éloignant de la partie d'insertion (2) ; et
un mécanisme de détection de déplacement (5a) configuré pour détecter un déplacement de la partie mobile (3) dans le corps,
**caractérisé par** :
une partie de perforation (3a) située sur une extrémité distale de la partie mobile (3) et configurée pour perforer un tissu péricardique,
le mécanisme de sollicitation (4) ayant une constante d'élasticité de telle sorte que le mécanisme de sollicitation (4) peut être comprimé contre sa force élastique lorsque la partie mobile est pressée contre un tissu corporel.

2. Dispositif de guidage (1) selon la revendication 1, dans lequel la partie de perforation est une partie de pointe aiguisée.

3. Dispositif de guidage (1) selon la revendication 1, dans lequel la partie de perforation est un foret.

4. Dispositif de guidage (1) selon la revendication 1, dans lequel la partie de perforation est une pince à dissection.

5. Dispositif de guidage (1) selon la revendication 1, dans lequel la partie de perforation est un couteau électrique.

6. Dispositif de guidage (1) selon la revendication 1, dans lequel la partie mobile (3) est disposée de façon à pouvoir se déplacer vers l'arrière et vers l'avant dans une direction longitudinale de la partie d'insertion (2),
le mécanisme de sollicitation (4) est configuré pour solliciter la partie mobile (3) dans une direction s'éloignant de l'extrémité distale de la partie d'insertion (2) dans la direction longitudinale de la partie d'insertion (2).

7. Dispositif de guidage (1) selon la revendication 6, dans lequel la partie mobile (3) comprend un mécanisme de direction configuré pour diriger l'extrémité distale de la partie mobile (3) dans une direction croisant la direction longitudinale à mesure que la partie mobile (3) fait saillie dans la direction longitudinale.

8. Dispositif de guidage (1) selon la revendication 1, dans lequel le mécanisme de détection de déplacement (5a) comprend un fil relié à la partie mobile (3) et ayant un repère disposé au niveau d'une position plus éloignée du côté proximal de la partie d'insertion (2) à l'extérieur de la partie d'insertion (2).

9. Dispositif de guidage (1) selon la revendication 1, dans lequel le mécanisme de détection de déplacement (5a) comprend au moins une partie de la partie mobile (3), la partie comprenant un matériau radio-opaque.

10. Dispositif de guidage (1) selon la revendication 1, comprenant en outre un conduit formé dans la direction longitudinale de la partie d'insertion (2) et ayant un orifice qui s'ouvre près de l'extrémité distale de la partie d'insertion (2) et un orifice d'injection qui s'ouvre sur le côté proximal de la partie d'insertion (2).
